Europäisches Patentamt

European Patent Office

Office européen des brevets

(11) Publication number: **0 234 826**
**A2**

## (12) EUROPEAN PATENT APPLICATION

(21) Application number: **87301298.3**

(22) Date of filing: **16.02.87**

(51) Int. Cl.³: **G 01 N 33/49**

(30) Priority: **18.02.86 US 830542**

(43) Date of publication of application:
**02.09.87 Bulletin 87/36**

(84) Designated Contracting States:
**BE DE FR GB IT**

(71) Applicant: **Ciba Corning Diagnostics Corp.
63 North Street
Medfield Massachusetts 02052(US)**

(72) Inventor: **Willis,Neil
Meadow Rise,Turnpike Close,Dinas Powys,
South Glamorgan,CF4 6HT,Wales(GB)**

(72) Inventor: **Mapleson,William W
One Padarn Close,
Cardiff,CF2 6ER Wales(GB)**

(74) Representative: **Froud, Clive
Elkington and Fife High Holborn House 52/54 High
Holborn
London WC1V 6SH(GB)**

(54) **Method and apparatus for measuring novel blood gas parameters.**

(57) The present invention relates to a method for mathematically generating an *in vivo* oxygen hemoglobin dissociation curve and novel blood gas parameters ($PaeO_2$, $PaEO_2$, $S_vO_{2x}$, $S_DO_2$, $P_x$ and $C_DO_2$) that can be derived therefrom. More particularly, measurements from an oxygen blood gas electrode and an oxygen Cooximeter are manipulated by a computer according to novel algorithms, such that the *in vivo* oxygen hemoglobin dissociation curve and the novel parameters are determined.

EP 0 234 826 A2

0234826

## METHOD AND APPARATUS FOR
## MEASURING NOVEL BLOOD GAS PARAMETERS

The present invention relates to a method for mathematically generating an _in vivo_ oxygen hemoglobin dissociation curve and novel blood gas parameters ($PaeO_2$, $PaEO_2$, $S_vO_{2x}$, $S_DO_2$, $P_x$, and $C_DO_2$) that can be derived therefrom. More particularly, measurements from an oxygen blood gas electrode and an oxygen Cooximeter are manipulated by a computer according to novel algorithms, such that the _in vivo_ oxygen oxyhemoglobin dissociation curve and the novel parameters are determined.

The measurement in arterial blood gases of the oxygen partial pressure ($PaO_2$), often referred to as the oxygen tension, is an accepted clinical parameter for determining the adequacy of the oxygen supply in a patient's blood. Various devices for measuring $PaO_2$ are commercially available, and their use is well documented in the literature. By assuming that the patient has a normal oxygen oxyhemoglobin dissociation curve (ODC), the clinician has used just the $PaO_2$ measurement for diagnosis of the available oxygen supply.

However, because many patients do not have a normal ODC, clinicians have also adopted the use of Cooximeters to determine oxygen saturation ($SaO_2$) in a patient's blood A discussion of oxygen saturation, oxygen content, and ODCs, can be found in an article by Neil Willis and Michael Clapham in _Clinica Chimica Acta,_ 150 (1985), 213-220.

The present invention relates to novel blood gas oxygen tension and saturation parameters that take into consideration the _in vivo_ affinity of a patient's hemoglobin for oxygen, and thus, links the traditional $PaO_2$ parameter

to a measured oxygen saturation reading. Methods and means for determining potential tissue supply from these parameters follow, but they are all based on a method of mathematically determining an _in vivo_ ODC from a single $PaO_2$ and $SaO_2$ measurement.

An _in vivo_ ODC can be mathematically generated from modifying an algorithm in accordance with a simultaneous pair of $PaO_2$ and $SaO_2$ measurements. Essentially the process is as follows:

a) one compares the relationship between measured $SaO_2$ and $PaO_2$ to those predicted by an algorithm which predicts a normal ODC having correction factors which are (or may become) known to affect ODC away from the standard or normal value (such as pH, temperature, 2,3 diphosphoglycerate 2,3-DPG, or $PCO_2$);

b) then one alters the algorithm by modifying any or all of the correction factors whereby the calculated $PaO_2/SaO_2$ relationship equals the measured one; and finally

c) one uses the modified algorithm to calculate an _in vivo_ ODC.

The first novel parameter is referred to as the "effective oxygen tension" $(PaeO_2)$. It is derived from measured $PaO_2$, measured $SaO_2$, and a calculated _in vivo_ ODC. More particularly, one performs the following steps:

a) measuring the $PaO_2$ and $SaO_2$ of the sample;

b) calculating the _in vivo_ ODC from the measured $PaO_2$ and $SaO_2$;

c) calculating the $SaO_2$ level on a normal ODC which corresponds to the measured $PaO_2$; and

d) calculating the $PaeO_2$ by finding the $PaO_2$ level on the _in vivo_ ODC which corresponds to the calculated $SaO_2$ level.

Another novel parameter is the "corrected effective oxygen tension" ($PaEO_2$). Although similar to $PaeO_2$, $PaEO_2$ is derived by additional calculations that assume a normal oxygen uptake. More particularly, one performs the following steps:

a) measuring the $PaO_2$ and $SaO_2$ of the sample;

b) calculating the <u>in vivo</u> ODC from the measured $PaO_2$ and $SaO_2$;

c) calculating the <u>in vivo</u> $SvO_2$, where the <u>in vivo</u> $SvO_2$ equals the measured $SaO_2$ minus 25%;

d) calculating the <u>in vivo</u> $pvO_2$ level on the <u>in vivo</u> ODC, where $pvO_2$ equals the $PO_2$ level which corresponds to the <u>in vivo</u> $SvO_2$;

e) calculating the $SvO_2$ level on the normal ODC which corresponds to the <u>in vivo</u> $pvO_2$;

f) calculating the effective $SaO_2$, where the effective $SaO_2$ equals the $SvO_2$ in e) above plus 25%; and

g) calculating the $PaEO_2$ by finding the $PO_2$ level on a normal ODC which corresponds to the effective $SaO_2$.

A third blood gas parameter is the "assumed mixed venous saturation" ($SvO_{2x}$) where X represents a $PO_2$ value in mm Hg. The steps for such a calculation are:

a) measuring the $PaO_2$ and $SaO_2$ of the sample;

b) calculating the <u>in vivo</u> ODC from the measured $PaO_2$ and $SaO_2$; and

c) calculating the $SvO_{2x}$ by finding the $SO_2$ level on the <u>in vivo</u> ODC that corresponds to a selected $PO_2$ level having X mm Hg.

A fourth blood gas parameter is the "potential oxygen saturation difference" ($S_DO_2$). Essentially, this is calculated by first determining $SvO_{2x}$ as above, then subtracting the $SvO_{2x}$ from the measured $SaO_2$.

A fifth blood gas parameter is the "potential oxygen content difference" ($C_DO_2$). Here, one uses the above $S_DO_2$ in the following manner:

$$C_DO_2 = \frac{S_DO_2 \times Hb \times 1.39}{100}$$

where Hb equals the total hemoglobin concentration of the sample in g/dl. For the first time, the clinician can be provided readily with how much oxygen is potentially being consumed by the tissue which can be related to a normal or reference range.

A sixth blood gas parameter is "oxygen tension at a high oxygen saturation" $P_X$, where X is between 90% and 100%. Here, the calculated O.D.C. is used to obtain the $PO_2$ at a high $SaO_2$ corresponding to X, preferably an $SaO_2$ of 95% and thus, a calculated parameter to be known as $P_{95}$. If the $PaO_2$ is greater than 95, i.e. the patient is on the plateau of the curve, enhanced oxygen therapy may not be beneficial.

All of these parameters can be determined by using standard measuring devices which measure $PaO_2$, such as blood gas analyzers, and $SaO_2$, such as Cooximeters. The necessary calculations can be done by using modified algorithms for normal oxyhemoglobin dissociation curves.

The advantage of these new parameters are most readily seen in the "marginal" oxygen therapy cases. For example, at the discriminating level for adjustment of oxygen therapy (60-70 mm Hg), the traditional $PaO_2$ values would indicate that therapeutical intervention could be needed. However, at this same level, because both $PaeO_2$ and $PaEO_2$ take the oxygen saturation levels into consideration, in many cases they would indicate that therapy can be discontinued. Increased inspired oxygen would be avoided along with, over time, the resultant damage to the epithelial elasticity and ciliary action of the lungs. Thus, in practice, the new parameters can permit patients to be removed safely from the costly and, if needlessly applied, damaging intensive care treatments of unnecessarily enhanced oxygen therapy in cases where the previous $PaO_2$ parameter would have contradicted.

Referring to the accompanying illustrative drawings:

FIGURE 1 is a graph of the human normal ODC.

FIGURE 2 is a graph of two abnormal human _in vivo_ ODC's and the normal human ODC.

FIGURE 3 is a graph comparing lactate/pyruvate ratios with $PaO_2$ and $PaeO_2$.

FIGURE 4 is a graph of how to calculate $PaeO_2$.

FIGURE 5 is a graph of how to calculate $PaEO_2$.

In order to measure the present parameters, one has to be able to generate oxyhemoglobin dissociation curves (ODCs) both for "normal" $paO_2/SaO_2$ values and actual patient $PaO_2$ and $SaO_2$ measurements. The algorithms needed for normal ODC curves are well known in the literature. (See L.J. Thomas Jr., Journal of Applied Physiology, 33, 1, July 1972, 154-158; and F.J.W. Roughton et. al., Journal of Applied Physiology, 35, 1973, 861-869. Given such an algorithm, it is well known to one of ordinary skill in the art how to generate a normal ODC, especially with the aid of a digital computer.

The algorithms needed for calculating _in vivo_ ODCs must contain correction factors descibed above. A representative algorithm is as follows:

$$SaO_2 = 100 \times \frac{a_1 + a_2 x^2 + a_3 x^3 + x^4}{a_4 + a_5 x + a_6 x^2 + a_7 x^3 + x^4}$$

where $a_1 = -8.522289$

$a_2 = 2.1214010 \times 10^3$

$a_3 = -6.7073989 \times 10^1$

$a_4 = 9.3596087 \times 10^5$

$a_5 = -3.1346258 \times 10^4$

$a_6 = 2.3961674 \times 10^3$

$a_7 = -6.7104406 \times 10^1$; and

$x = PaO_2 \times 10^{-y}$; and

$y = 0.024 \, (37-temp) + 0.40 \, (pH-7.40) + 0.06 \, (\log 40 - \log PCO_2)$.

While the above calculations can be done manually, preferably, one will use a programmable digital microprocessor. Moreover, the above algorithm is intended to be representative of any known or hereafter developed algorithms for normal ODCs. The ordinarily skilled artisan could extend the above principles to any such suitable algorithms.

Of course, when one speaks of a normal ODC, there must be an accepted set of normal pH, $PCO_2$ and temperature values for the blood being analysed. In humans, at pH 7.4, $PCO_2 = 40$ mmHg and 37°C, the normal ODC is such that at $SaO_2 = 50\%$ $PaO_2 = 26.5$ mmHg (3.53 KPa).

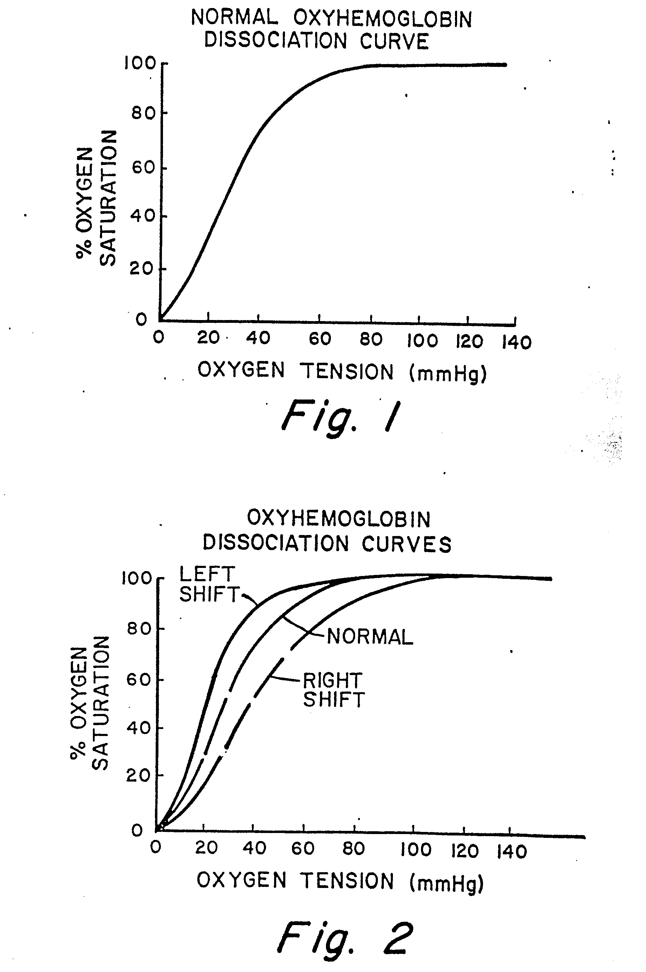

Whereas the normal ODC is a fixed standard, in vivo ODCs can be shifted left or right in response to physiological factors. FIGURE 2 shows left and right shifted in vivo ODCs. An explanation of such shifts can be found in Corning Medical/Technical Bulletin #2015 published December, 1983.

Comparison of $PaO_2$ with $PaeO_2$ and $PaEO_2$

Heparinised blood samples were taken from patients for routine blood gas analysis who were previously analyzed to have a $PaO_2$ below 80 mm Hg. Two ml of the blood samples was placed immediately after withdrawal into a polypropylene tube containing Long's reagent and mixed vigorously. (See Long, C., _Biochemistry Journal_, 38, 1944, 447).

Blood gas analysis was carried out using an ABL 2 blood gas analyzer (Radiometer, Copenhagen) for pH, $pCO_2$, $pO_2$, standard bicarbonate, and base excess and a Cooximeter M2500 (Corning Medical, Halstead, Essex) for hemoglobin oxygen saturation and oxygen content. Lactate and pyruvate was measured enzymatically using a modified method of Hadjivassilion employing auto analyzer equipment (Technicon, Basingstoke) and a fluorimeter (Locarte, London).

The data obtained from 49 samples were used to plot graphs of:

- Lactate/pyruvate ratio against $PaO_2$ and $PaeO_2$

- Lactate/pyruvate ratio against $PaO_2$ and $PaEO_2$

- Arterial oxygen content against $PaO_2$ and $PaeO_2$

- Arterial oxygen content against $PaO_2$ and $PaEO_2$

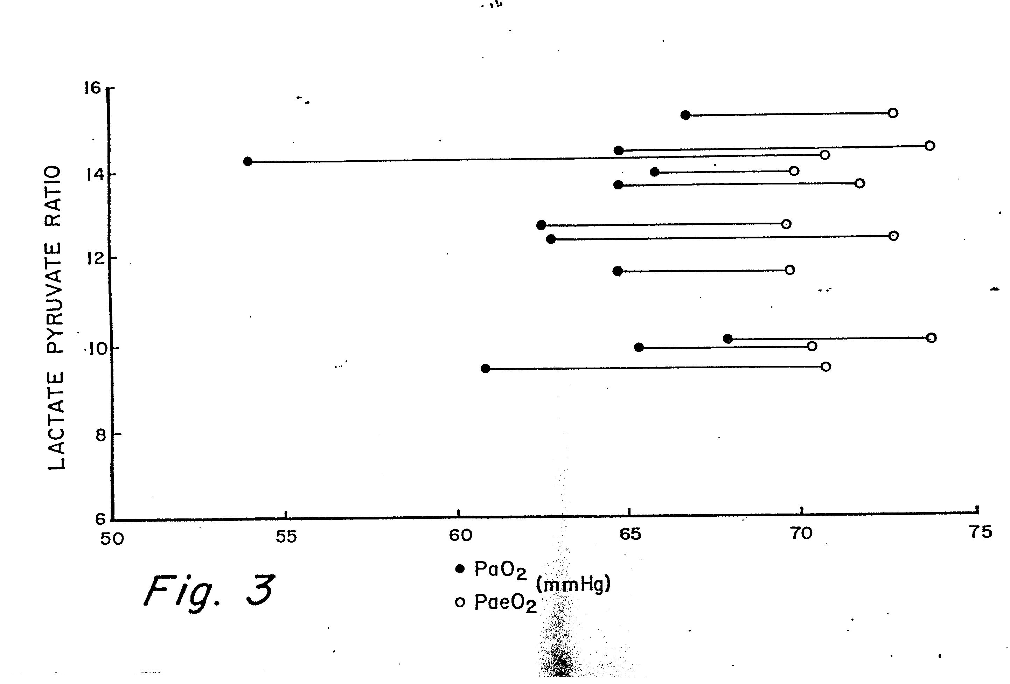

Eleven patients with a lactate/pyruvate ratio less than 15, considered to be normal for post-operative cardiopulmonary bypass patients, were compared with the respective $PaO_2$ and $PaeO_2$ values (FIGURE 3).

The $PaeO_2$ and $PaEO_2$ at fixed $PaO_2$ and $PvO_2$ were plotted against the theoretical $S_DO_2$ values obtained from a series of ODC's.

The relationship between $PaO_2$, $PaeO_2$ or $PaEO_2$, and the arterial oxygen content all follow an upward trend. Low levels of $PaO_2$, $PaeO_2$, and $PaEO_2$ all predict high lactate/pyruvate levels but at the discriminating level for oxygen therapy adjustment (between 60-70mmHg), $PaeO_2$ and $PaEO_2$ predict lactate/pyruvate levels in the normal range far better than $PaO_2$. This fact is highlighted when the borderline $PaO_2$ values between 60-70 are plotted.

Preferably, the calculations necessary to determine the present parameters are done on a digital microprocessor. The choice of computer hardware and software capable of this is within the skill of the ordinary artisan. However, for demonstration purposes the calculation of $PaeO_2$ and $PaEO_2$ shall be demonstrated graphically below.

## PeO2

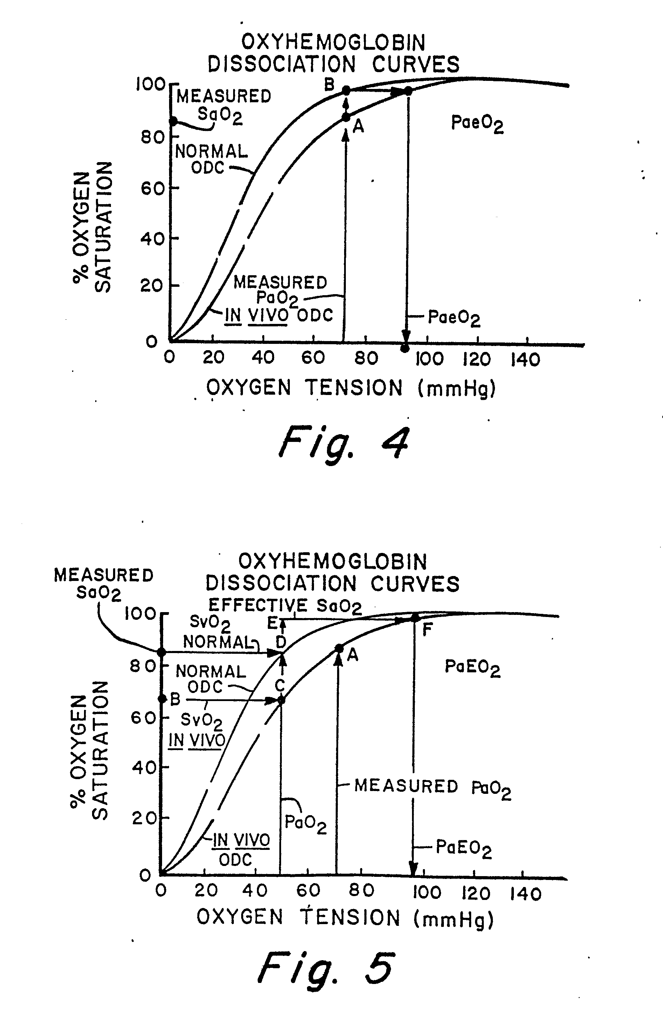

FIGURE 4 graphically depicts how to calculate $PaeO_2$. Assuming that the measured $PaO_2$ and $SaO_2$ are, respectively, 70 mmHg and 86% oxygen saturation, then using the above algorithm one would generate the _in vivo_ ODC as depicted at point A. One calculates the calculated $SaO_2$ level on the normal ODC which corresponds to 70 mmHg point B. Finally, one goes back to the _in vivo_ ODC at the calculated $SaO_2$ level (to point C) and reads down to find the $PaeO_2$ at point D.

## PaEO2

FIGURE 5 graphically depicts how to calculate $PaEO_2$. As in the $PaeO_2$ example, one generates the _in vivo_ ODC. Next, the _in vivo_ venous oxygen saturation is calculated as depicted at point B ($SvO_2 = SaO_2$ minus 25%). One then goes to the _in vivo_ ODC and determines the corresponding $PvO_2$ level (point C). Using the $PvO_2$ level one calculates the calculated $SvO_2$ level using the normal ODC (point D). Now one adds 25% onto the calculated $SvO_2$ to get the effective $SaO_2$ (point E).

Finally, one goes back to the _in vivo_ ODC at the effective $SaO_2$ level and reads down to find the $PaEO_2$ at point F.

The following Table demonstrates clearly how deceiving the traditional $PaO_2$ value can be for a patient at the discriminating level of 60 mm Hg.

## TABLE

| Patient | $PaO_2$ * | $SaO_2$ + | $Hb$ ** | $PaeO_2$ * | $S_DO_2$ + | $C_DO_2$ ++ |
|---------|-----------|-----------|---------|------------|------------|-------------|
| A | 60 | 85 | 17 | 73 | 46.7 | 11.05 |
| B | 60 | 92 | 17 | 58 | 39.5 | 9.35 |
| C | 60 | 92 | 10 | 58 | 39.5 | 5.50 |

\* mm Hg

\*\*  g/dl

\+ %

++ ml/dl ($PvO_2$ = 27 mm)

Whereas the $PaO_2$ indicates all of the patients are the same, the $PaeO_2$ tells a different story. Patient A does not need oxygen therapy because the $PaeO_2$ is over 70 mm Hg, moreover he has a healthy $C_DO_2$ (>9). Patient B may need therapy because his $PaeO_2$ is less than 60 but he has a high Hb of 17. However, at least he is in better shape than Patient C. Not only is Patient C's $PaeO_2$ 60, but his $C_DO_2$ is dangerously low at 5.5. Normal or reference ranges for $C_DO_2$ will be established in the field according to existing practices.

Quite dramatic differences in the condition of these three patients are missed completely by $PaO_2$. Only with the aid of the present parameters would a clinician be able to remove from therapy one who is receiving needless, expensive, and damaging treatment (Patient A), and to spot the one who is in great immediate danger of dying (Patient C).

# Claims:

1. A method for determining a novel blood gas parameter ($PaeO_2$) of a blood sample comprising:

    a) measuring the $PaO_2$ and the $SaO_2$ of the sample;

    b) calculating the _in vivo_ ODC;

    c) calculating the $SaO_2$ level on a normal ODC which corresponds to the measured $PaO_2$; and

    d) calculating the $PaeO_2$ by finding the $PaO_2$ level on the _in vivo_ ODC which corresponds to the calculated $SaO_2$ level.

2. The method of claim 1 wherein the $PaO_2$ and $SaO_2$ are measured simultaneously.

3. An apparatus for determining a novel blood gas parameter ($PaeO_2$) of a blood sample comprising:

    a) means for measuring the $PaO_2$ of the sample;

    b) means for measuring the $SaO_2$ of the sample;

    c) means for calculating the _in vivo_ ODC;

    d) means for calculating the $SaO_2$ level on a normal ODC which corresponds to the measured $PaO_2$; and

    e) means for calculating the $PaeO_2$ by finding the $PO_2$ level on the _in vivo_ ODC which corresponds to the calculated $SaO_2$ level.

4. The apparatus of claim 3 wherein the measuring means of a) and b) are combined into one sampling device.

5. The apparatus of claim 3 wherein the calculating means of c), d), and e) are combined into a digital microprocessor.

6. The apparatus of claim 4 incorporating the calculating means of claim 5.

7. A method for determining a novel blood gas parameter ($PaEO_2$) of a blood sample comprising:

    a)    measuring the $PaO_2$ and the $SaO_2$ of the sample;

    b)    calculating the in vivo ODC;

    c)    calculating the in vivo $SvO_2$, where in vivo $SvO_2$ equals the measured $SaO_2$ minus 25%;

    d)    calculating the in vivo $PvO_2$ level, where in vivo $PvO_2$ equals the $PO_2$ level on the in vivo ODC which corresponds to the in vivo $SvO_2$;

    e)    calculating the calculated $SvO_2$ level on a normal ODC which corresponds to the in vivo $PvO_2$;

    f)    calculating the effective $SaO_2$, where the effective $SaO_2$ equals the calculated $SvO_2$ plus 25%; and

    g)    calculating $PaEO_2$, the level on the in vivo ODC which corresponds to the effective $SaO_2$.

8. The method of claim 7 wherein the $PaO_2$ and $SaO_2$ are measured simultaneously.

9. An apparatus for determining a novel blood gas parameter ($PaEO_2$) of a blood sample comprising:

    a)    means for measuring the $PaO_2$ of the sample;

    b)    means for measuring the $SaO_2$ of the sample;

c) means for calculating the <u>in vivo</u> $SvO_2$, where the <u>in vivo</u> $SvO_2$ of the measured $SaO_2$ minus 25%;

d) means for calculating the <u>in vivo</u> ODC;

e) means for calculating the <u>in vivo</u> $PvO_2$ level on the <u>in vivo</u> ODC, where $PvO_2$ equals the $PO_2$ level which corresponds to the <u>in vivo</u> $SvO_2$;

f) means for calculating the calculated $SvO_2$ level on a normal ODC which corresponds to the <u>in vivo</u> $PvO_2$;

g) means for calculating the effective $SaO_2$, where the effective $SaO_2$ equals the calculated $SvO_2$ plus 25%; and

h) means for calculating $PaEO_2$, the level on the normal <u>in vivo</u> ODC which corresponds to the effective $SaO_2$.

10. The apparatus of claim 9 wherein the measuring means of a) and b) are combined into one sampling device.

11. The apparatus of claim 9 wherein the calculating means of c) - h) are combined into a digital microprocessor.

12. The apparatus of claim 10 incorporating the calculating means of claim 11.

13. A method for determining a novel blood gas parameter ($SvO_{2x}$) of a blood sample comprising:

a) measuring the $PaO_2$ and the $SaO_2$ of the sample;

b) calculating the <u>in vivo</u> ODC; and

c) calculating the $SvO_{2x}$ by finding the $SO_2$ level on the <u>in vivo</u> ODC that corresponds to a selected $PO_2$ level of x, where x is between 20 and 50 mm Hg.

14. The method of claim 13 wherein the $PaO_2$ and $SaO_2$ are measured simultaneously.

15. An apparatus for determining a novel blood gas parameter ($SvO_{2x}$) of a blood sample comprising:

   a) means for measuring the $PaO_2$ of the sample;

   b) means for measuring the $SaO_2$ of the sample;

   c) means for calculating the *in vivo* ODC; and

   d) means for calculating the $SvO_{2x}$ by finding the $SO_2$ level on the *in vivo* ODC that corresponds to a selected $PO_2$ level of x, where x is between 20 and 50 mmHg.

16. The apparatus of claim 15 wherein the measuring means of a) and b) are combined into one sampling device.

17. The apparatus of claim 15 wherein the calculating means of c) and d) are combined into a digital microprocessor.

18. The apparatus of claim 16 incorporating the calculating means of claim 17.

19. A method for determining a novel blood gas parameter ($S_DO_2$) comprising:

   a) measuring the $PaO_2$ and $SaO_2$;

   b) calculating the $SvO_{2x}$; and

   c) calculating the $S_DO_2$ by subtracting the $SvO_{2x}$ from the measured $SaO_2$.

20. The method of claim 19 wherein the $PaO_2$ and $SaO_2$ are measured simultaneously.

21. An apparatus for determining a novel blood gas parameter ($S_DO_2$) of a blood sample comprising:

a) means for measuring the $PaO_2$ of the sample;

b) means for measuring the $SaO_2$ of the sample;

c) means for calculating the $SvO_{2x}$; and

d) means for calculating the $S_DO_2$ by subtracting the $SvO_2$ from the measured $SaO_2$.

22. The apparatus of claim 21 wherein the measuring means of a) and b) are combined into one sampling device.

23. The apparatus of claim 21 wherein the calculating means of c) and d) are combined into a digital microprocessor.

24. The apparatus of claim 22 incorporating the calculating means of claim 23.

25. A method for generating the _in vivo_ ODC of a blood sample comprising:

a) measuring the $PaO_2$ and $SaO_2$ of a blood sample;

b) comparing the relationship between the measured $PaO_2$ and $SaO_2$ to that calculated by an algorithm for a normal ODC having at least one correction factor;

c) modifying the standard value of at least one correction factor, whereby the calculated $PaO_2$ and $SaO_2$ relationship equals the measured relationship; and

d) calculating further $PaO_2$ and $SaO_2$ relationships using the algorithm with any of the modified correction factors.

26.    The method of claim 25 wherein the correction factors are selected from the group consisting of pH, temperature, 2,3 diphosphoglycerate levels, and $PCO_2$.

27.    An apparatus for generating the _in vivo_ ODC of a blood sample comprising:

a)    means for measuring the $PaO_2$ and $SaO_2$ of a blood sample;

b)    means for comparing the relationship between the measured $PaO_2$ and $SaO_2$ to that calculated by an algorithm for a normal ODC having at least one correction factor;

c)    means for modifying the standard value of at least one correction factor, whereby the calculated $PaO_2$ and $SaO_2$ relationship equals the measured relationship; and

d)    means for calculating further $PaO_2$ and $SaO_2$ relationships using the algorithm with any of the modified correction factors.

28.    A method for determining a novel blood gas parameter ($P_X$), where X is between 90 and 100, comprising:

a)    measuring the $PaO_2$ and $SaO_2$ of the sample;

b)    calculating the _in vivo_ ODC; and

c)    calculating the $P_X$ by finding the $PO_2$ level on the _in vivo_ ODC that corresponds to a selected $SaO_2$ level of X%.

29.    An apparatus for determining a novel blood gas parameter ($P_X$), where X is between 90 and 100, comprising:

a)    means for measuring the $PaO_2$ and $SaO_2$ of the sample;

b)    means for calculating the _in vivo_ ODC; and

0234826

c)  means for calculating the $P_X$ by finding the $PO_2$ level on the <u>in</u> <u>vivo</u> ODC
   that corresponds to a selected $SaO_2$ level of X%.

NORMAL OXYHEMOGLOBIN
DISSOCIATION CURVE

Fig. 1

OXYHEMOGLOBIN
DISSOCIATION CURVES

Fig. 2

Fig. 3

0234826

3/7

Fig. 4

Fig. 5